# EUROPEAN PATENT APPLICATION

(11) **EP 4 737 473 A1**
(43) Date of publication of application: **06.05.2026**
(21) Application number: 24830622.7
(22) Date of filing: 20.06.2024
(51) Int. Cl.: C07K 7/08, A61K 38/10, A61P 27/02

(54) **NOVEL COMPLEMENT INHIBITOR AND PREPARATION, USE AND PRODUCT THEREOF**

(30) Priority: 29.06.2023 CN 202310781313
(71) Applicant: Liu, Xiaorong, Nanjing, Jiangsu 210033 (CN)
(72) Inventor: DAI, Peng, Nanjing, Jiangsu 210032 (CN); WANG, Ye, Nanjing, Jiangsu 210032 (CN)
(74) Representative: Winter, Brandl - Partnerschaft mbB
(86) International application number: PCT/CN2024/100500
(87) International publication number: WO 2025/001976

(57) **Abstract**

Provided in the present invention are a novel complement inhibitor, and a preparation, the use and a product thereof, which belong to the field of molecular medicine. The portion having complement inhibitory activity comprises a cyclic peptide and a pharmaceutically acceptable salt thereof. The cyclic peptide is shown as general formula I: R1-ICXaa₃-Xaa₄-QD-Xaa₇-G-Xaa₉-HRCT-R2, wherein Xaa₇ and Xaa₉ are selected from uncommon amino acids, and Xaa₃ and Xaa₄ include at least one uncommon amino acid. When the cyclic peptide provided in the present invention is used as an active ingredient of the complement inhibitor, the cyclic peptide has good affinity to a C3 protein, has good pharmacokinetic properties, and has good application prospects.

## Description

### TECHNICAL FIELD

The disclosure belongs to the field of molecular medicine, and specifically to novel complement inhibitor and preparation, application and products thereof.

### BACKGROUND ART

Complement refers to a group of globulins with enzyme-like activity after activation, present in the blood, tissue fluid, and cell membrane surfaces of humans and animals. The complement system is an important component of innate immunity and participates in immune regulation and immune damaging reactions in the human body. Composed of more than 30 soluble proteins and membrane-bound proteins, the complement system has three main activation pathways: the classical pathway, the alternative pathway, and the lectin activation pathway. Complement exists in an inactive state in plasma and exhibits biological activity upon activation. It can eliminate immune complexes through cytolysis, opsonization, phagocytosis, and mediation of inflammatory responses, thereby exerting corresponding biological functions.

The three activation pathways of the complement system c are centered around the formation of C3 convertase and C5 convertase. By cleaving C3 and C5 to produce corresponding bioactive fragments, the complement response is further amplified. These fragments modify the target surface and can promote phagocytosis, inflammation, immune regulation, and other processes. Among them, the process from contact with the activating agent to the generation of C3 convertase (and cleavage of C3) can be regarded as the front-end reactions of these activation pathways. Although the front-end reactions of these activation pathways are different, they share a common terminal pathway, i.e., the generation of C5 convertase. C5 is cleaved to form C5b fragments, which sequentially react with C6, C7, C8, and C9 to ultimately form the membrane attack complex (MAC), exerting a cytolytic effect.

Although the complement system plays an important role in human immunity, inappropriate or excessive complement activation is the root cause or contributing factor of many serious diseases. The discovery of complement inhibitors has provided a promising research direction for the treatment of related diseases. Complement drugs have been approved for marketing in diseases such as paroxysmal nocturnal hemoglobinuria (PNH) and geographic atrophy caused by age-related macular degeneration. As known from the three activation pathways of the complement system, inhibiting C3 can control the early reactions of the activation pathways, so the development of C3 complement inhibitors has broad application prospects. As the most abundant complement protein in human serum, C3 consists of α and β chains. Activated C3 is cleaved into fragments with important biological activities: C3a, C3b, iC3a, C3d, etc. C3 contains 41 exons and has an abundant structure providing multiple different ligand-binding sites, such as the conserved domains disclosed in the prior art: α2-macroglobulin domain (A2M), C3a anaphylatoxin domain, etc. (Zan Qi, Liu Xin, Pang Yue, et al., Research progress of complement C3 structure and function [J], China Journal of Immunology, 2014(04): 549-553, DOI:10.3969/j.issn.1000-484X.2014.04.031.), which are important in immune surveillance and immune response pathways.

Compstatin is the first discovered C3 complement inhibitor, a thirteen-residue cyclic peptide that can bind both C3a and C3b and prevent the cleavage of native C3 by C3 convertase. This cyclic peptide is a molecule composed of a polar part and a non-polar part. The polar part includes a type I β-turn, and the non-polar part includes a disulfide bond. The prior art has verified that the four residues of the β-turn and the disulfide bond of the surrounding hydrophobic cluster play important roles in the inhibitory activity of C3 complement inhibitor. Based on the initially disclosed compstatin structure, the prior art has continuously optimized it to improve its pharmaceutical properties. For example, the optimized compstatin analog Ac-ICVW(CH₃)QDWGAHRCT-NH2 (C-C) has an activity 16 times that of the parent, as recorded in "Morikis D, Soulika A M, Mallik B, et al. Improvement of the anti-C3 activity of compstatin using rational and combinatorial approaches".

Empaveli (pegcetacoplan) is the first marketed C3 complement inhibitor drug, a synthetic cyclic peptide conjugated to a polyethylene glycol polymer that specifically binds C3a and C3b. Currently, Empaveli has been approved for indications including paroxysmal nocturnal hemoglobinuria (PNH) and geographic atrophy caused by dry macular degeneration.

Other optimization methods of compstatin disclosed in the prior art further include methylation on the peptide backbone and substitution at flanking positions, leading to the development of a series of compstatin analogs with improved potency (Qu et al., 2011, Molecular Immunology 48: 481-489; WO2010/127336). These modifications have resulted in compstatin analogs exhibiting binding affinity superior to that of most active analogs reported to date.

Chinese Patent Application No. CN201980030725.6 discloses a compstatin analog with C-terminal and/or N-terminal modifications, which improves the solubility of the peptide without significantly reducing its pharmacokinetic properties, and in some cases, confers enhanced plasma and vitreous stability and/or binding affinity for C3 and its fragments. However, it does not actually improve the properties of the cyclic peptide itself.

There are also some optimization methods that involve optimizing the type of amino acids in the peptide segment. However, there are various ways to optimize amino acids, and the results of any optimization method are not deterministic. Given the therapeutic potential of compstatin analogs in various diseases, it is quite important to further optimize compstatin to obtain greater potency.

### SUMMARY

To solve the aforementioned problems, the disclosure provides a novel complement inhibitor and preparation, application and products thereof.

### Terms:

In the disclosure, "complement inhibitor" is sometimes referred to as complement inhibitory analog. A complement inhibitor is a cyclic peptide that binds to the complement component C3 and inhibits complement activation. Complement inhibitors inhibit the cleavage of C3 into C3a and C3b by convertases. Since C3 is a central component of all three complement activation pathways, complement inhibitors and their analogs can inhibit the activation of proteins that converge all three pathways. The ability of complement inhibitors and their analogs to inhibit the alternative pathway of complement activation can significantly promote their efficacy in certain ocular conditions described herein.

In the disclosure, a "cyclic peptide" generally refers to a cyclic polypeptide, i.e., a polypeptide chain formed by chemical bonds between amino acids (protein or non-protein amino acids) in a cyclic sequence. Cyclization can generally be achieved through disulfide bonds, amide bonds, or other cyclization methods disclosed in the prior art. Multiple cyclization methods may exist in the same cyclic peptide.

In the disclosure, "polypeptide" refers to a polymer of amino acids, optionally including one or more amino acid analogs. A protein is a molecule composed of one or more polypeptides. A peptide is a relatively short polypeptide, usually about 2 to 60 amino acids in length. The terms "protein", "polypeptide", and "peptide" are used interchangeably. Polypeptides as used herein may contain amino acids, such as those naturally occurring in proteins, those not naturally occurring in proteins, and/or non-amino acid amino acid analogs. As used herein, an amino acid "analog" may be a different amino acid structurally similar to an amino acid, or a compound other than an amino acid structurally similar to an amino acid. A large number of industry-recognized analogs of the 20 amino acids commonly found in proteins are known. One or more amino acids in a polypeptide may be modified, for example, by adding chemical entities (such as carbohydrate groups, phosphate groups, farnesyl groups, geranylgeranyl groups, fatty acid groups, conjugate linkers), functionalization, or other modifications.

In the disclosure, the abbreviations are defined as follows:

| Abbreviation | Name | Molecular Formula |
|---|---|---|
| Pip | 4-Aminopiper | C₆H₁₂N₂O₂ |
| 2Nal | 2-Naphthyl-alanine | C₁₃H₁₃NO₂ |
| 5F-Trp | 5-Fluoro-DL-tryptophan | C₁₁H₁₁FN₂O₂ |
| Aib | 2-Aminoisobutyric acid | C₄H₉NO₂ |
| Abu | 2-Aminobutyric acid | C₄H₉NO₂ |
| Bpa | Benzoyl phenylalanine | C₁₆H₁₅NO₃ |
| AzaTrp | azatryptophan | C₁₀H₁₁N₃O₂ |
| W(CH₃) | 1-Methyl-L-tryptophan | C₁₂H₁₄N₂O₂ |

In the disclosure, abbreviations not specifically indicated refer to current general abbreviations, specifically, the abbreviations for common amino acids.

In the disclosure, "uncommon amino acids" include all types of amino acids except the 20 common amino acids known in the art, including natural amino acids and unnatural amino acids. Natural amino acids may be those naturally occurring in nature that have been discovered or not yet discovered. Unnatural amino acids may be artificially modified amino acids.

In the disclosure, methods for artificially modifying "unnatural amino acids" include, but are not limited to: phosphorylation, methylation, acetylation, ubiquitination, glycosylation, etc. Artificially modified amino acids prepared by conventional amino acid modification methods in the art are all within the scope of unnatural amino acids covered by the disclosure.

The disclosure first provides a novel complement inhibitor.

The active ingredient of complement inhibitor comprises a cyclic peptide or a pharmaceutically acceptable salt thereof, wherein the cyclic peptide is shown in the general formula I;
general formula I: R1-IC-Xaa₃-Xaa₄-QD-Xaa₇-G-Xaa₉-HRCT-R2 (C-C);
wherein Xaa₇ and Xaa₉ are selected from uncommon amino acids and derivatives thereof; at least one of Xaa₃ and Xaa₄ is an uncommon amino acid or a derivative thereof; and R1 and R2 are modifying groups.

Specifically, Xaa₃ and Xaa₄ may each independently include one uncommon amino acid; or Xaa₃ may be selected from uncommon amino acids and Xaa₄ may be selected from common amino acids; or Xaa₄ may be selected from uncommon amino acids and Xaa₃ may be selected from common amino acids.

The uncommon amino acids in the Formula may be: tryptophan (W) analogs, alanine (A) analogs, aminobutyric acid analogs, aminoisobutyric acid analogs, 4-amino-4-piperidinecarboxylic acid analogs, or benzoylphenylalanine analogs.

Modifications of the analogs may include methylation, acetylation, hydroxylation, carboxylation, phosphorylation, naphthylation, nitrogen atom hybridization, fluorination, acylation, alkoxylation, or halogenation.

Preferably, the tryptophan analog is selected from: 5-fluoro-DL-tryptophan, azatryptophan, or N-methyltryptophan.

Preferably, when Xaa₃ is selected from uncommon amino acids, it may be Abu;

Preferably, when Xaa₃ is selected from common amino acids, it may be V or Y.

Preferably, when Xaa₄ is selected from uncommon amino acids, it may be any one of 2Nal, W(CH₃), or Bpa.

In some preferred embodiments, Xaa₄ must be selected from uncommon amino acids.

Preferably, Xaa₇ is selected from either 2Nal or 5F-Trp.

Preferably, Xaa₉ is selected from either Aib or Pip.

In some specific embodiments, Xaa₃, Xaa₄, Xaa₇, and Xaa₉ are each independently selected from the above preferred options, including but not limited to:
(1) In the cyclic peptide, Xaa₃ is selected from V or Y, and Xaa₄ is selected from any one of 2Nal, W(CH₃), or Bpa;
or (2) In the cyclic peptide, Xaa₄ is selected from any one of 2Nal, W(CH₃), or Bpa, and Xaa₇ is selected from either 2Nal or 5F-Trp;
or (3) In the cyclic peptide, Xaa₄ is selected from any one of 2Nal, W(CH₃), or Bpa, and Xaa₉ is selected from either Aib or Pip;
or (4) In the cyclic peptide, Xaa₃ is Abu, and Xaa₇ is selected from either 2Nal or 5F-Trp;
or (5) In the cyclic peptide, Xaa₃ is Abu, and Xaa₉ is selected from either Aib or Pip;
or (6) In the cyclic peptide, Xaa₃ is Abu, Xaa₄ is selected from any one of 2Nal, W(CH₃), or Bpa, Xaa₇ is selected from either 2Nal or 5F-Trp, and Xaa₉ is selected from either Aib or Pip;
or (7) In the cyclic peptide, Xaa₃ is selected from V or Y, Xaa₄ is selected from any one of 2Nal, W(CH₃), or Bpa, Xaa₇ is selected from either 2Nal or 5F-Trp, and Xaa₉ is selected from either Aib or Pip.

R1 or R2 may be selected from reactive functional groups, including but not limited to the following groups: alkenes, acetylene, alcohols, phenols, ethers, oxides, halides, aldehydes, ketones, carboxylic acids, esters, amides, cyanates, isocyanates, thiocyanates, isothiocyanates, amines, hydrazines, hydrazones, acylhydrazines, diazonium groups, nitro groups, nitriles, thiols, sulfides, disulfides, sulfoxides, sulfones, sulfonic acids, sulfuric acids, acetals, ketals, anhydrides, sulfates, hydrogen thioperoxide, isonitriles, amidines, imides, imidoesters, nitrones, hydroxylamines, oximes, hydroxamic acids, thiohydroxamic acids, allenes, ortho-esters, sulfites, enamines, ynamines, ureas, pseudoureas, aminomocovina, carbodiimides, carbamates, imines, azides, azo compounds, azoxy compounds, and nitroso compounds. Reactive functional groups also include groups often used in the preparation of bioconjugates, such as N-hydroxysuccinimide esters, maleimides, thiols, and the like.

Preferably, R1 may include one or more of hydrogen, acetyl, methoxy, PEG, or carboxyl.

Preferably, R2 may include amino.

Preferably, R1 or R2 includes an amino acid or a variant thereof.

In some embodiments, R1 or R2 independently includes tyrosine or isoleucine.

In some embodiments, R1 includes isoleucine and R2 includes tyrosine.

Preferably, R1 and R2 form an amide bond to cyclize the polypeptide head-to-tail.

In some embodiments, the active ingredient of the complement inhibitor is selected from any one or more of SEQ ID NO.1-6:
SEQ ID NO.1: Ac-ICV-W(CH₃)-QD-2Nal -G-Aib-HRCT-NH₂ (C-C) ;
SEQ ID NO.2: Ac-ICV-AzaTrp-QD-5F-Trp-G-Aib-HRCT-NH₂ (C-C) ;
SEQ ID NO.3: Ac-ICV-2Nal-QD-5F-Trp-G-Aib-HRCT-NH₂ (C-C) ;
SEQ ID NO.4: Ac-IC-Abu-W(CH₃)-QD-5F-Trp -G-Pip-HRCT-NH₂ (C-C) ;
SEQ ID NO.5: Ac-IC-Abu- 2Nal -QD-5F-Trp -G-Aib-HRCT-NH₂ (C-C) ;
SEQ ID NO.6: Ac-IC-Y-Bpa-QD-5F-Trp -G-Aib-HRCT-NH₂ (C-C) .

Under certain conditions, general formula I has a structure similar to compstatin:
Cyclization is achieved through a disulfide bridge formed between Cys-2 and Cys-12. Cys-2 refers to the cysteine (C) at position 2 in the general formula, and Cys-12 refers to the cysteine (C) at position 12 in the general formula
The complement inhibitor may directly include any one or more of the aforementioned cyclic peptides;
The complement inhibitor may include products obtained by side chain modification of the aforementioned cyclic peptides, and the side chain modification may be small molecule protein modification, C-terminal modification.

In the disclosure, "side chain modification" may be referred to as "lateral chain modification" or "branch chain modification" in some cases. As known to those skilled in the art, the terminals of a peptide are defaulted to a free amino group at the N-terminus and a free carboxyl group at the C-terminus. The terminals of peptides often require capping, i.e., acetylation at the N-terminus and amidation at the C-terminus. This modification avoids introducing excess charges, enhances resistance to exopeptidase degradation, and improves peptide stability.

In the disclosure, small molecule proteins used for modification may exert their modifying effect through covalent modification. The covalent modifications may be inorganic small molecules or organic small molecules, such as phosphorylation, glycosylation, acylation, and the like.

The complement inhibitor may also be an optimized product of the aforementioned general formula I obtained by conventional technical means without changing the activity of the cyclic peptide itself. The optimization may occur on R1 or R2, or on the amino acids of the main chain. Examples of optimization methods include PEGylation, conjugation, homo-multimerization, hetero-multimerization, amino acid chirality optimization, incorporation of hydrophilic/charged residues, addition of cell-penetrating peptides, and in-chain N-methylation. For example, preferably, Xaa9 may include N-methyl derivatives. General formula I or its optimized form may be regarded as a compstatin analog.

In the disclosure, "methylation" refers to the process of catalyzing the transfer of a methyl group from an active methyl compound to another compound, which can form various methylated compounds, or refers to the chemical modification of certain proteins to form methylated products. Specifically, it refers to the chemical modification of the cyclic peptides provided by the disclosure to form methylated products.

In the disclosure, "in-chain N-methylation" refers to methylation occurring on any one or more amino acids in the chain, specifically, it refers to the process of attaching one methyl group to the -NH₂ group of any one or more amino acids in the chain to form an -NH-CH₃ structure. Preferably, the in-chain N-methylation occurs on any one in-chain amino acid. In some embodiments, the N-methylation occurs on the 9th amino acid of the cyclic peptide described in the disclosure

In the disclosure, "amino acid chirality" refers to molecules with a certain configuration or conformation that are not identical to their mirror images and cannot overlap with each other. Chirality is used to express the relationship between optically active molecules and their mirror images that cannot overlap. All chiral molecules have optical activity, and all compounds with optical activity are chiral molecules. Chiral molecules include asymmetric molecules without any symmetry elements and dissymmetric molecules with a simple axis of symmetry but no other symmetry elements.

The complement inhibitor may be general formula I with an added cell-penetrating peptide. The cell-penetrating peptide may be a cell-penetrating peptide disclosed in the prior art, or an amino acid sequence not yet disclosed in the prior art but still performing the same function as cell-penetrating peptides in the prior art.

As recognized by those skilled in the art, cell-penetrating peptides (CPPs), also known as transduction peptides, are a class of short peptide molecules capable of penetrating cell membranes. They can enter cells through mechanisms such as membrane perturbation and membrane inversion and interact with target molecules. Cell-penetrating peptides can play a role in drug delivery. Cell-penetrating peptides are usually composed of 3-30 amino acids and have characteristics such as positive charge or hydrophobicity. These properties allow them to interact with negatively charged components on the cell membrane (such as phosphatidylinositol) and enter the cell interior. Cell-penetrating peptides also have good biological stability, repeatable synthesis, and modifiability. In the disclosure, the cell-penetrating peptide used to optimize general formula I to obtain the complement inhibitor is not limited to a specific type. It may be composed of 3-30 amino acids, or a number of amino acids outside this range. Generally, the cell-penetrating peptide may have a length of 2-10, 2-12, 2-18, 2-20, 2-25, 5-10, 5-20, 5-25, 10-15, 10-20, 10-25, 15-20, 15-25, 15-30, 20-30, 25-30, 13-19, or 18-24 amino acids. In other embodiments, the cell-penetrating peptide may have modifications, such as the cell-penetrating peptide TAT: H2N-YGRKKRRQRRR-OH.

Incorporation of hydrophilic/charged residues can be used as a method to increase the solubility of cyclic peptides. Therefore, the complement inhibitor may be general formula I with incorporated hydrophilic/charged residues, and the number of residues may be two or three. The incorporation of hydrophilic/charged residues may occur at the C-terminus, N-terminus, or both. Specifically, in general formula I, it may occur on R1, R2, or both. "Both" means that both R1 and R2 include the incorporation of hydrophilic/charged residues. In some cases, it can be considered that the solubility of the peptide increases with the increase in the number of lysine residues, but this does not mean that this modification has the same definite effect in all cases. Residues that can be incorporated into general formula I include KK, KKK, KRK, RRR. In some cases, adding two or three lysine residues can improve the solubility of the cyclic peptide, and while maintaining inhibitory ability, improve binding affinity, exhibit improved pharmacokinetic characteristics, expand the range of administration routes, reduce the frequency of administration during chronic treatment, and improve drug compliance. This has been disclosed in some prior art, such as "Nadja B, Tchilabalo A, Resuello R, et al. New analogs of the complement C3 inhibitor compstatin with increased solubility and improved pharmacokinetic profile[J]. Journal of Medicinal Chemistry, 2018:acs.jmedchem.8b00560.".

Preferably, the incorporation of hydrophilic/charged residues occurs on R2, specifically, connected before the amino group. More specifically, any one or more K or R are connected before the amino group. Optionally, one K or R may be connected; KK, RR, KR, or RK may be connected; or three amino acid residues may be connected, where each amino acid residue is independently K or R, such as any one of KKK, KKR, KRK, RKK, KRR, RKR, RRK, or RRR.

The complement inhibitor may include a clearance-reducing moiety with a molecular weight of 1 kD to 100 kD on the basis of the cyclic peptide of the aforementioned general formula I. Specifically, the clearance-reducing moiety includes polyethylene glycol (PEG). This optimization does not affect the inhibitory activity of the cyclic peptide itself and is a conventional optimization method in the art.

Preferably, the molecular weight of the clearance-reducing moiety may be 20-80 kD, 20-90 kD, 30-80 kD, 30-90 kD, 40-80 kD, 40-50 kD, 50-60 kD, 70-80 kD, 70-90 kD, or 80-90 kD.

Preferably, the PEG may be branched PEG or linear PEG; more preferably, the clearance-reducing moiety includes branched PEG with 3-10 branches, and at least about 50% of the branches have a compstatin analog moiety attached thereto.

Furthermore, the clearance-reducing moiety includes branched PEG with 3-4, 3-6, 3-9, 4-5, 4-7, 4-9, 5-6, 5-8, 6-7, 6-9, 7-8, 7-9, or 8-9 branches; at least about 55%, 58%, 60%, 64%, 65%, 70%, 75%, 76%, 80%, 85%, 90%, 98%, or 95% of the branches have a compstatin analog moiety attached thereto.

By adding molecules (such as polyethylene glycol or similar molecules), the optionally attached cell-reactive moiety or targeting moiety can be modified to stabilize the compound, reduce its immunogenicity, extend its lifespan in vivo, enhance or reduce its solubility, and/or enhance its resistance to degradation. PEGylation methods are well known in the art. PEGylation is the process of covalently conjugating activated PEG to protein or polypeptide molecules through chemical methods. The complement inhibitor may be PEGylated at the C-terminus, N-terminus, or both. Specifically, polyethylene glycol molecules are added to R1 or R2 of general formula I, or to both R1 and R2. Alternatively, as disclosed in the prior art, low molecular weight PEG chains are attached to the C-terminus or Lys residues of compstatin to develop new analogs of the C3 inhibitor compstatin. Compared with the parent peptide, conjugation with 3 kD PEG can significantly improve solubility at physiological pH, maintain inhibitory activity, and show a better pharmacokinetic profile. PEGylation is a clinically mature half-life extension technology, and its safety has been fully verified in years of clinical practice and has been approved by drug regulatory authorities in most countries/regions for intravenous, oral, and dermal use in humans.

In some embodiments, as known to those skilled in the art, modifying the terminal regions of the cyclic peptide can enhance the solubility of the peptide, such as PEG modification, but it is not limited to PEG modification. Under certain conditions, those skilled in the art can know that in some cases, the size of the PEG chain is directly related to the solubility of the compound, but this does not mean that this is the case in all cases, and other modifications may affect the solubility of the compound.

Preferably, the PEG modification occurs on R1 and/or R2, and may also include any one or both of carboxyl and methoxy. The PEG modification can be freely combined with carboxyl or methoxy.

Preferably, R2 of general formula I after PEG modification may have a structure similar to the following: -COCH₂-PEG-OMe; the molecular weight of PEG refers to that described for the clearance-reducing moiety, which may be 1 kD to 100 kD.

In another embodiment, the cyclic peptide is fused to the Fc domain of an immunoglobulin or a portion thereof. In some other embodiments, the cyclic peptide is conjugated to an albumin moiety or an albumin-binding peptide. Thus, in certain embodiments, the cyclic peptide is modified with one or more polypeptide or non-polypeptide components, for example, to pegylate the cyclic peptide or conjugate it to another moiety. In certain embodiments, the component is not the Fc domain of an immunoglobulin or a portion thereof. The cyclic peptide may be provided as part of a multimer or supramolecular complex, which may include a single molecular substance or multiple different substances.

In certain embodiments, the complement inhibitor is a multivalent compound comprising a plurality of cyclic peptides covalently or non-covalently linked to a polymeric backbone or polymeric scaffold. Each cyclic peptide connected may be the same or different. In certain embodiments of the disclosure, the cyclic peptide comprises multiple instances or copies of a single compstatin analog moiety. In other embodiments of the disclosure, the multivalent compound comprises one or more instances of each of two or more different cyclic peptides (e.g., 3, 4, 5, or more different cyclic peptides). In certain embodiments of the disclosure, the number of cyclic peptides may be 1-10. In other embodiments of the disclosure, the number of cyclic peptides may be 10-20, 20-100, 100-1000, 1000-10000, 10000 to 50000, 50000-100000, 100000 to 1000000.

The cyclic peptide may be directly linked to the polymeric scaffold, or may be linked via a linking moiety that connects the cyclic peptide to the polymeric scaffold. The linking moiety may be connected to a single cyclic peptide and to the polymeric scaffold. Alternatively, the linking moiety may have multiple cyclic peptides attached thereto, such that the linking moiety connects the multiple cyclic peptides to the polymeric scaffold.

In certain embodiments, the cyclic peptide comprises an amino acid with a side chain containing a primary amines or secondary amine, such as a Lys residue. For example, a Lys residue or an amino acid sequence containing a Lys residue is appended to the N-terminus and/or C-terminus of the cyclic peptide. In certain embodiments, the cyclic moiety of the Lys cyclic peptide is separated by a rigid or flexible spacer. The spacer may, for example, comprise a substituted or unsubstituted, saturated or unsaturated alkyl chain, an oligo(ethylene glycol) chain, and/or other moieties. The length of the chain may be, for example, 2-20 carbon atoms. In other embodiments, the spacer is a peptide. The length of the peptide spacer may be, for example, 1-20 amino acids, such as 4-20 amino acids in length. For example, a suitable spacer may comprise or consist of multiple Gly residues, Ser residues, or both. Optionally, at least one amino acid in the amino acid with a side chain containing a primary amine or secondary amine and/or the spacer is a D-amino acid. Any of a variety of polymeric backbones or scaffolds may be used. For example, the polymeric backbone or polymeric scaffold may be a polyamide, polysaccharide, polyanhydride, polyacrylamide, polymethacrylate, polypeptide, polyethylene oxide, or dendrimer. In one embodiment, the polymeric backbone or polymeric scaffold comprises a variety of reactive functional groups, such as carboxylic acids, anhydrides, or succinimide groups. The polymeric backbone or scaffold reacts with the cyclic peptide. In one embodiment, the cyclic peptide comprises any of a variety of different reactive functional groups (such as carboxylic acids, anhydrides, or succinimide groups) that react with appropriate groups on the polymeric backbone. Alternatively, monomer units that can bind to each other to form a polymeric backbone or scaffold are first reacted with the cyclic peptide, and the resulting monomers are polymerized. In another embodiment, short chains are prepolymerized, functionalized, and then a mixture of short chains of different compositions is assembled into a longer polymer.

In some specific embodiments, the complement inhibitor comprises a cell-reactive functional group that reacts with a thiol (SH) group to form a covalent bond.

In some specific embodiments, the complement inhibitor comprises a cell-reactive functional group that reacts with an amine group to form a covalent bond.

The cyclic peptide in the disclosure, as the active ingredient of the complement inhibitor, is mainly used to inhibit the activation of C3 complement.

The disclosure also provides a method for preparing the complement inhibitor according to the disclosure based on the amino acid sequence.

The cyclic peptides of the disclosure can be prepared by condensation of one or more amino acid residues according to conventional peptide synthesis methods through various peptide synthesis methods. Other methods for synthesizing peptides or peptidomimetics by solid-phase or liquid-phase methods are well known to those skilled in the art. During peptide synthesis, the side chain amino and carboxyl groups may be protected/deprotected as needed using commonly known protecting groups. Modification of selective peptides and peptide derivatives with protecting groups is obvious to those skilled in the art.

Alternatively, certain peptides of the disclosure can be produced by expression in appropriate prokaryotic or eukaryotic systems. For example, a DNA construct can be inserted into a plasmid vector suitable for expression in bacterial cells (such as Escherichia coli) or yeast cells (such as Saccharomyces cerevisiae), or into a baculovirus vector for expression in insect cells or a viral vector for expression in mammalian cells. Such vectors contain regulatory elements required for the expression of DNA in host cells, arranged in a manner that allows the expression of DNA in host cells. Such regulatory elements required for expression include promoter sequences, transcription initiation sequences, and optionally enhancer sequences.

SEQ ID NO.1-6 and other similarly designed peptides are suitable for production by in vitro or in vivo expression of nucleic acid molecules. A DNA construct encoding a peptide concatemer can be introduced into an in vivo expression system, and the upper limit of the concatemer depends on the expression system used. After the concatemer is produced, cleavage between the C-terminal Asn and the following N-terminal G is achieved by exposing the polypeptide to hydrazine.

Peptides produced by expressing genes in recombinant prokaryotic or eukaryotic systems can be purified according to methods known in the art.

In one embodiment, a commercially available expression/secretion system can be used, whereby the recombinant peptide is expressed and then secreted from the host cell, facilitating purification from the surrounding medium.

A combination of gene expression and synthetic methods can also be used to produce the aforementioned cyclic peptides. For example, they can be produced by gene expression and thereafter subjected to one or more post-translational synthetic processes, such as modification of the N- or C-terminus or cyclization of the molecule.

Secondly, the disclosure provides the use of the aforementioned complement inhibitor in the preparation of a medicament.

The complement inhibitor is used for the treatment of complement-mediated disorders, specifically C3 complement-mediated disorders. Specifically, it inhibits the cleavage of C3 into C3a and C3b by C3 convertase.

Diseases targeted by the medicament include, but are not limited to: any one or combination of: ophthalmic diseases, glomerulonephritis, systemic lupus erythematosus, Streptococcus pneumoniae infection, skin rashes, inflammation, paroxysmal nocturnal hemoglobinuria (PNH), atypical haemolytic uraemic syndrome (aHUS), complement-mediated hemolysis, adult respiratory distress syndrome, heart diseases, and complement-mediated injury;
The medicament can also play a protective or therapeutic role in hemodialysis (Mastellos, D. C. et al., Compstatin: a C3-targeted complement inhibitor reaching its prime for bedside intervention.), kidney transplantation (Mastellos, D. C. et al., Complement C3-targeted therapy: replacing long-held assertions with evidence-based discovery.), and hemorrhagic shock (van Griensven, M et al. Protective effects of the complement inhibitor compstatin Cp40 in hemorrhagic shock.), as disclosed in the prior art.

The inflammation includes, but is not limited to, acute inflammation or chronic inflammation. In some embodiments, the inflammation manifests as periodontitis. Periodontitis is a prevalent chronic oral disease caused by excessive inflammation induced by an unfavorable biofilm microbial community formed in the subgingival tooth sites, and may lead to tooth loss, impaired chewing, and nutritional status. The incidence of periodontitis is high. Early clinical and histological observations of human periodontitis indicate that periodontal inflammation and tissue destruction are associated with increased complement activity. Therefore, the medicament of the disclosure can be used for the treatment of periodontitis by exerting the effect of a complement inhibitor.

The complement-mediated injury includes, but is not limited to: any one or combination of early tissue injury in infectious diseases, tumor rejection, ischemia/reperfusion (I/R) injury, and rejection after xenotransplantation.

The ocular diseases include, but are not limited to: any one or combination of macular degeneration, choroidal neovascularization, retinal neovascularization, ocular inflammation, and geographic atrophy.

In some cases, the diseases targeted by the medicament may also be: atypical haemolytic uraemic syndrome (aHUS); dense deposit disease (DDD); C3 glomerulonephritis (C3GN); C3 glomerulopathy; complement-mediated nephropathy and glomerulonephritis; age-related macular degeneration (AMD); ophthalmic diseases characterized by macular degeneration, choroidal neovascularization (CNV), retinal neovascularization (RNV), proliferative vitreoretinopathy, glaucoma, uveitis, ocular inflammation, or any combination thereof; paroxysmal nocturnal hemoglobinuria (PNH); cold agglutinin disease (CAD); warm antibody autoimmune hemolytic anemia (wAIHAs); sickle cell disease; transplant-associated thrombotic microangiopathy; rheumatoid arthritis (RA); systemic lupus erythematosus(SLE); autoimmune and autoinflammatory kidney diseases; autoimmune myocarditis; multiple sclerosis; traumatic brain and spinal cord injury; cerebral, intestinal, and renal ischemia-reperfusion (IR) injury; spontaneous and recurrent abortion; antiphospholipid syndrome (APS); Parkinson's disease; Alzheimer's disease; neurodegenerative inflammatory diseases supported by abnormal synaptic remodeling, microglial activity, and cognitive decline; asthma; antineutrophil cytoplasmic antibody-associated vasculitis (Wegener's syndrome); non-lupus autoimmune skin diseases, such as pemphigus, bullous pemphigoid, and epidermolysis bullosa; post-traumatic shock; cancer; gingivitis; and atherosclerosis.

The disclosure also provides the use of the aforementioned complement inhibitor in the treatment of the above diseases.

When the complement inhibitor is used for treatment, its dosage can range from milligram to gram level. For different indications, the dosage can be adjusted. For example, when applied locally for indications, the dosage can be set at the milligram level; when applied systemically for indications, the dosage can be set at the gram level. The local indications include, for example, the aforementioned ocular diseases or periodontitis; the systemic indications include, for example, paroxysmal nocturnal hemoglobinuria, atypical hemolytic uremic syndrome, etc. The above are only applicable methods selectable according to general treatment methods, and do not mean that the complement inhibitor of the disclosure can only be administered in the above ways.

For the delivery of the composition or agent of the disclosure, "local administration" or "local delivery" refers to delivery that does not rely on the transport of the composition or agent to its intended target tissue or site through the vascular system. The composition or agent can be directly delivered to its intended target tissue or site, or to the vicinity thereof, for example, delivered immediately adjacent to the intended target tissue or site. For example, the composition or agent can be delivered by injection or implantation of the composition or agent, or by injection or implantation of a device containing the composition or agent. After local administration near the target tissue or site, the composition or agent or one or more of its components can diffuse to the intended target tissue or site. It should be understood that after completing local delivery, a portion (usually only a small portion of the administered dose) of the therapeutic agent may enter the vascular system and be transported to another location, including back to its intended target tissue or site.

Furthermore, the disclosure provides a medicament comprising the aforementioned complement inhibitor.

The medicament includes any one or more of the aforementioned complement inhibitors or pharmaceutically acceptable salts thereof.

Tandem or multimers of the complement inhibitor or its salt can also be used in the disclosure.

In some cases, the medicament includes any one or more of SEQ ID NO.1-6 or modifications thereof.

The medicament may also include other pharmaceutically acceptable carriers or excipients.

The pharmaceutically acceptable carriers or excipients include, but are not limited to: buffers, excipients, stabilizers, preservatives, fragrances, flavoring agents, membrane penetration enhancers, enzyme inhibitors, pH regulators, or other excipients that do not affect the activity of the inhibitor but may affect the druggability.

The dosage form of the medicament includes, but is not limited to, injections and infusions. Those skilled in the art can select the drug medicament form according to the specific indication, which is not limited to the content disclosed in the disclosure, and may also be medicament dosage forms disclosed or not disclosed in the prior art.

The medicament can be administered by subcutaneous injection or intravenous injection.

The administration method of the medicament can be local injection, including but not limited to: transvascular, transdermal, transocular, or intraperitoneal injection.

The administration method of the medicament can also be other methods capable of achieving therapeutic effects, such as transdermal or oral administration.

Preferably, the medicament may be an ophthalmic preparation, preferably an intravitreal injection or an eye drop preparation.

As used herein, "pharmaceutically acceptable salts" refer to derivatives of the disclosed compounds, wherein the parent compound is modified by preparing its acid or base salts. Examples of pharmaceutically acceptable salts include, but are not limited to: inorganic or organic acid salts of basic residues such as amines; basic or organic salts of acidic residues such as carboxylic acids; and the like. Pharmaceutically acceptable salts include conventional salts or quaternary ammonium salts of the parent compound formed from inorganic or organic acids. Such conventional salts include, but are not limited to: salts derived from inorganic acids such as hydrochloric acid, hydrobromic acid, sulfuric acid, sulfamic acid, phosphoric acid, nitric acid, etc.; and salts prepared from organic acids such as acetic acid, propionic acid, succinic acid, glycolic acid, stearic acid, lactic acid, malic acid, tartaric acid, citric acid, ascorbic acid, palmitic acid, maleic acid, hydroxymaleic acid, phenylacetic acid, glutamic acid, benzoic acid, salicylic acid, sulfanilic acid, 2-acetoxybenzoic acid, fumaric acid, p-toluenesulfonic acid, methanesulfonic acid, ethanedisulfonic acid, oxalic acid, isethionic acid, etc. Certain acidic or basic compounds of the disclosure may exist in zwitterionic form. All forms of the compounds, including free acids, free bases, and zwitterions, are included within the scope of the disclosure.

As used herein, "treatment" refers to providing therapy, i.e., administering any type of medical or surgical treatment to a subject. The purpose of providing treatment may be to reverse, alleviate, or inhibit the development of a disease, disorder, or condition, prevent or reduce the likelihood of its occurrence, or reverse, alleviate, inhibit, or prevent one or more symptoms or phenomena of a disease, disorder, or condition, prevent or reduce the likelihood of its occurrence. "Prevention" refers to preventing the occurrence of a disease, disorder, condition, or its symptoms or phenomena in at least some individuals for at least a period of time. Treatment may include administering an agent to a subject after the occurrence of one or more symptoms or phenomena indicating a condition (such as macular degeneration or diabetic retinopathy) for the purpose of (for example) reversing, alleviating, reducing the severity of the condition, and/or inhibiting or preventing the development of the condition, and/or reversing, alleviating, reducing the severity of one or more symptoms or manifestations of the condition, and/or inhibiting one or more symptoms or phenomena of the condition. The composition of the disclosure can be administered to a subject who has developed an ocular disease (such as exudative or non-exudative ARMD or diabetic retinopathy) or who is at an increased risk of developing such a condition compared to members of the general population. The composition of the disclosure can be administered prophylactically, i.e., before the occurrence of any symptoms or phenomena of the condition. Usually, in such cases, the subject is at risk of developing the condition.

Beneficial effects of the disclosure:
When the cyclic peptide provided by the disclosure is used as the active ingredient of a complement inhibitor, it has good affinity for C3 protein and inhibitory activity. When used as a medicament, the complement inhibitor of the disclosure has better pharmacokinetic properties such as in vivo stability and solubility, improves compliance, and has a wide range of clinical applications.

In the disclosure, "in vivo stability" refers to the property of a medicament to maintain its own structure and properties unchanged by resisting biological activities such as enzymatic degradation in vivo. It is generally expressed as the time during which the radiopharmaceutical maintains its basic properties in animal or human serum.

In the disclosure, "compliance" (Patient compliance/Treatment compliance), also known as obedience or conformity, refers to the behavior of a patient following the doctor's instructions for treatment, which is customarily called patient "cooperation"; otherwise, it is called non-compliance. Compliance can be divided into three categories: complete compliance, partial compliance (excessive or insufficient dosage, increased or decreased frequency of administration, etc.), and complete non-compliance. In actual treatment, each of these three types of compliance accounts for 1/3. The compliance of a patient with a specific medication is the compliance of that specific medicament

In the disclosure, "pharmacokinetics", mainly studies the dynamic changes of the body's disposition of drugs. It includes the processes of absorption, distribution, biochemical transformation (or metabolism), and excretion of drugs in the body, especially the law of blood drug concentration changing with time. The metabolism of drugs is related to factors such as human age, gender, individual differences, and genetic factors.

### BRIEF DESCRIPTION OF THE DRAWINGS

FIG. 1: the mass spectrum of SEQ ID NO.1.
FIG. 2: the mass spectrum of SEQ ID NO.2.
FIG. 3: the mass spectrum of SEQ ID NO.3.
FIG. 4: the mass spectrum of SEQ ID NO.4.
FIG. 5: the mass spectrum of SEQ ID NO.5.
FIG. 6: the mass spectrum of SEQ ID NO.6.
FIG. 7: the HPLC chromatogram of SEQ ID NO.1.
FIG. 8: the HPLC chromatogram of SEQ ID NO.2.
FIG. 9: the HPLC chromatogram of SEQ ID NO.3.
FIG. 10: the HPLC chromatogram of SEQ ID NO.4.
FIG. 11: the HPLC chromatogram of SEQ ID NO.5.
FIG. 12: the HPLC chromatogram of SEQ ID NO.6.
FIG. 13: the real-time binding curve of the sample in the BLI affinity assay.
FIG. 14: the fitting curve of Sample 2 in the MST affinity assay.
FIG. 15: the fitting curve of the positive control sample in the MST affinity assay.

### DETAILED DESCRIPTION

The disclosure will be further described in detail below with reference to specific embodiments. The following embodiments are not intended to limit the disclosure but are only used to illustrate the disclosure. The experimental methods used in the following embodiments are conventional methods unless otherwise specified. For experimental methods without specific conditions, they are usually carried out according to conventional conditions. The materials, reagents, etc. used in the following embodiments are commercially available unless otherwise specified.

### Example 1

In this example, the following cyclic peptides were prepared as the active ingredients of the complement inhibitor:
SEQ ID NO.1: Ac-ICV-W(CH₃)-QD-2Nal -G-Aib-HRCT-NH₂(C-C);
SEQ ID NO.2: Ac-ICV-AzaTrp-QD-5F-Trp-G-Aib-HRCT-NH₂(C-C);
SEQ ID NO.3: Ac-ICV-2Nal-QD-5F-Trp-G-Aib-HRCT-NH₂(C-C);
SEQ ID NO.4: Ac-IC-Abu-W(CH₃)-QD-5F-Trp -G-Pip-HRCT-NH₂(C-C);
SEQ ID NO.5: Ac-IC-Abu- 2Nal -QD-5F-Trp -G-Aib-HRCT-NH₂(C-C);
SEQ ID NO.6: Ac-IC-Tyr-Bpa-QD-5F-Trp -G-Aib-HRCT-NH₂(C-C).
Cyclization is achieved through a disulfide bridge formed between Cys-2 and Cys-12. Cys-2 refers to the cysteine (C) at position 2 in the formula, and Cys-12 refers to the cysteine (C) at position 12 in the formula.

SEQ ID NO.1-6 in this example were synthesized by F-moc solid-phase peptide synthesis and purified by reversed-phase high-performance liquid chromatography (RP-HPLC) according to methods known in the art. However, the disclosure is not limited to their specific preparation methods. Any product obtained by preparing the above sequences or modifying the above sequences through preparation methods disclosed or not disclosed in the prior art is within the scope of the disclosure. Those skilled in the art are capable of performing conventional technical optimizations on the above active ingredients and verifying various activities of the optimized cyclic peptides, such as the activities shown in the experimental examples, but not limited to the data forms presented in the experimental examples.

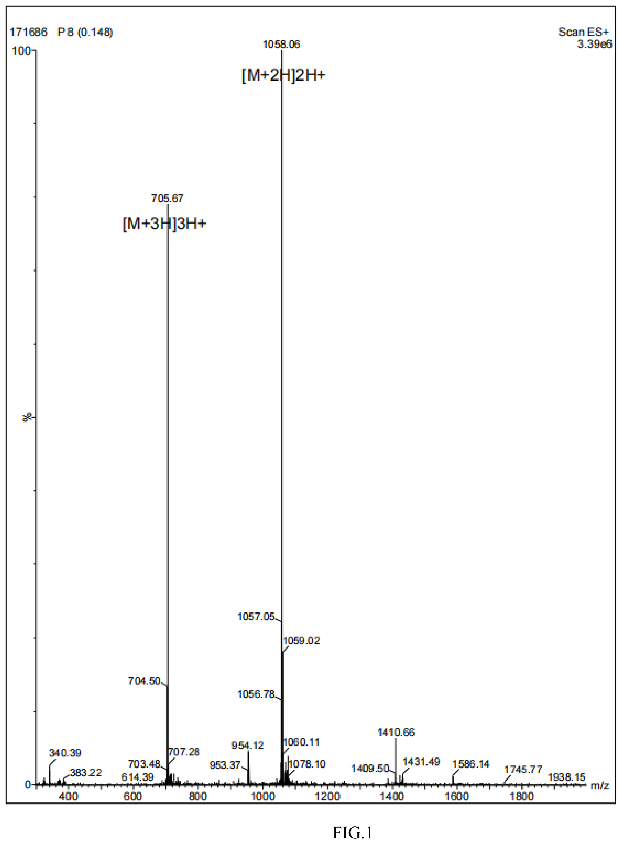

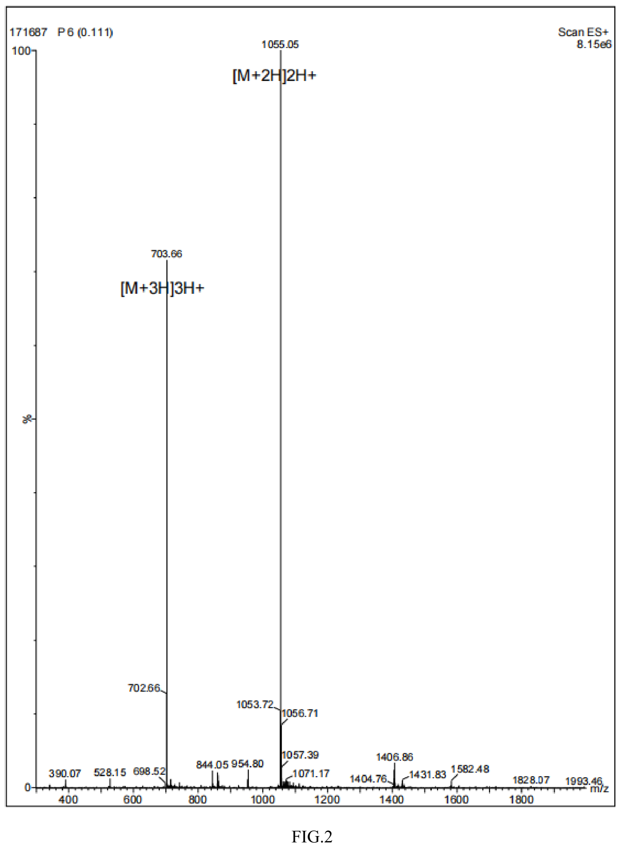

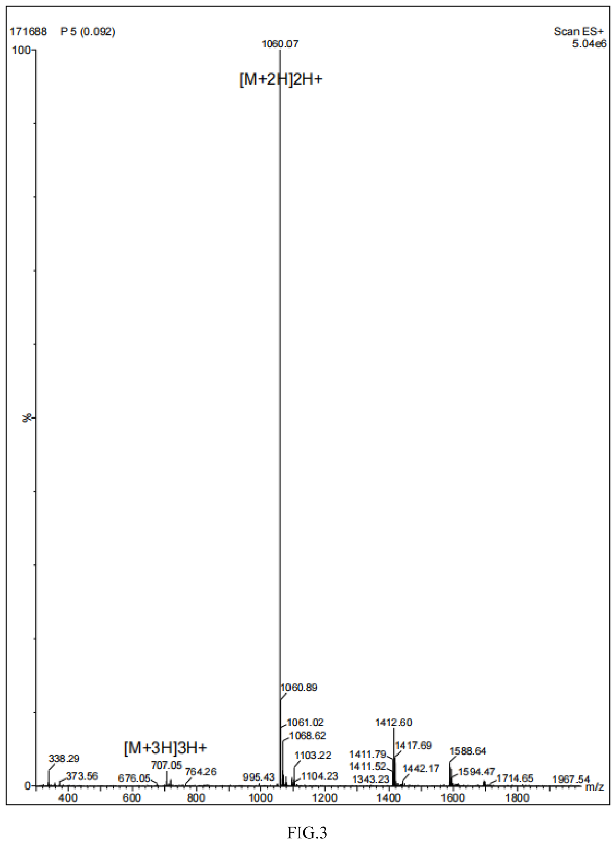

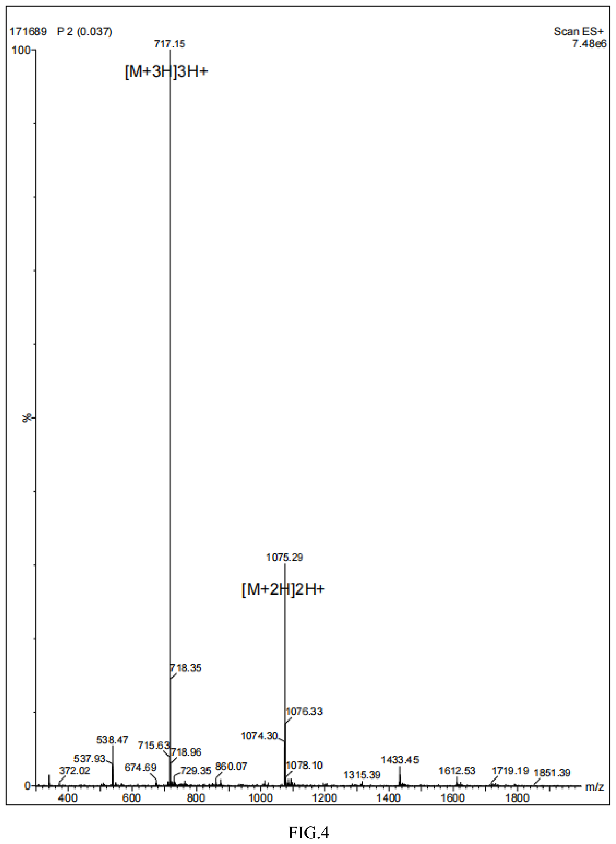

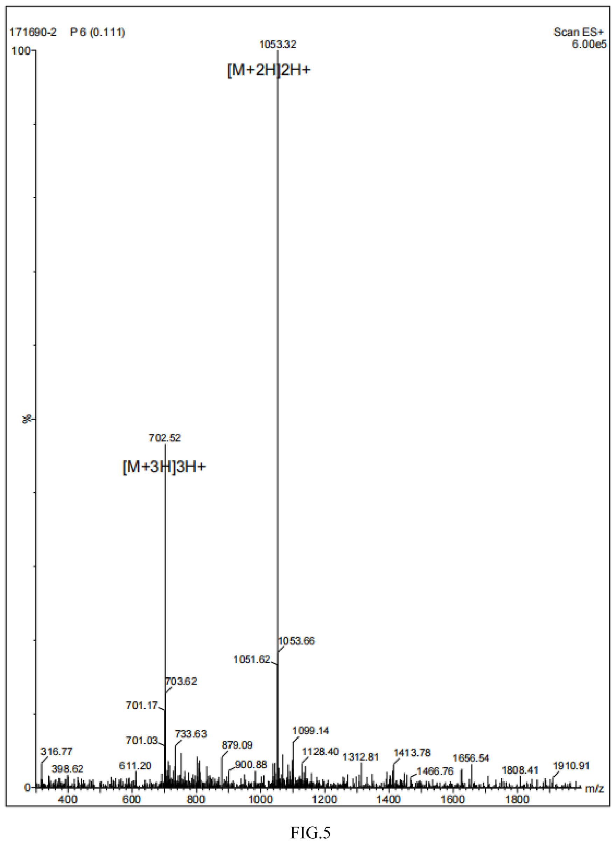

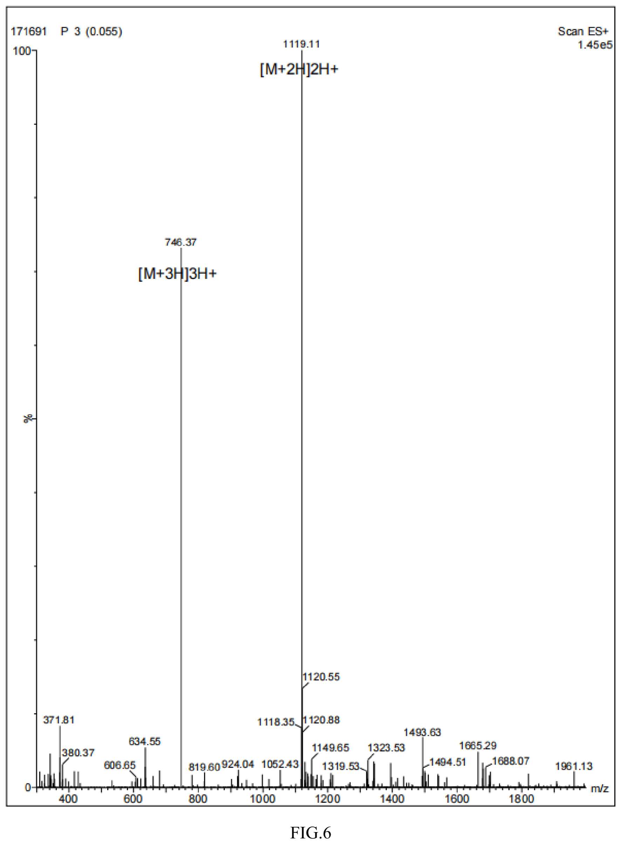

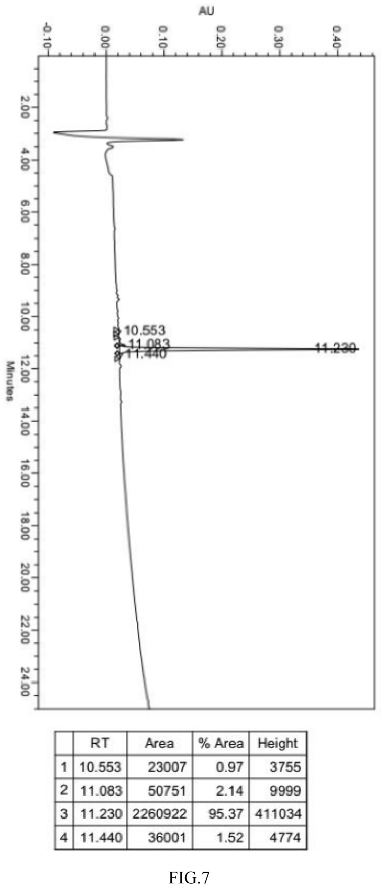

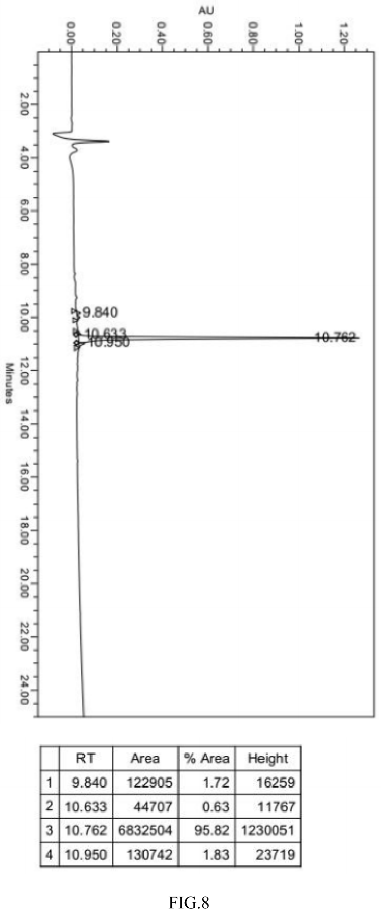

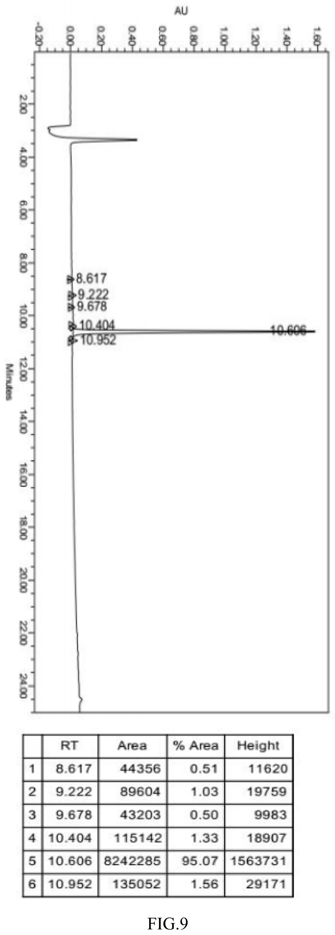

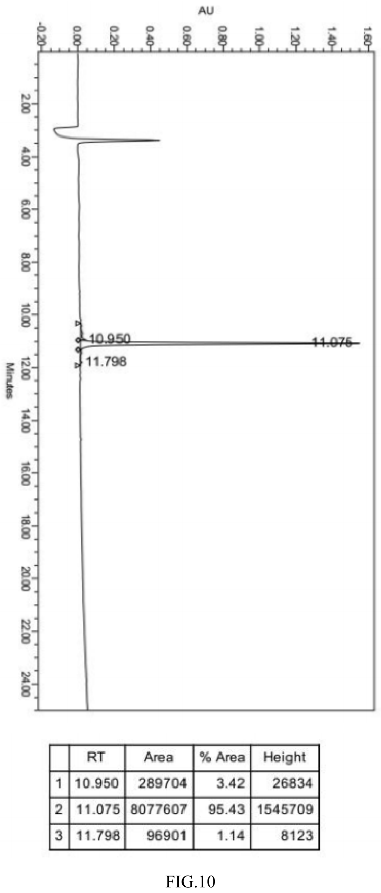

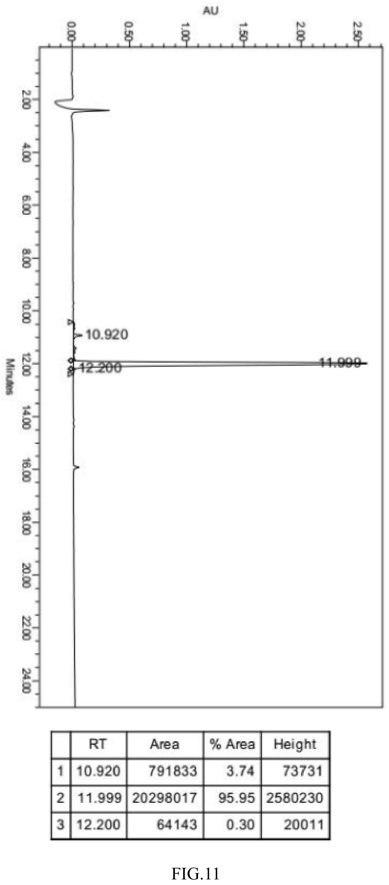

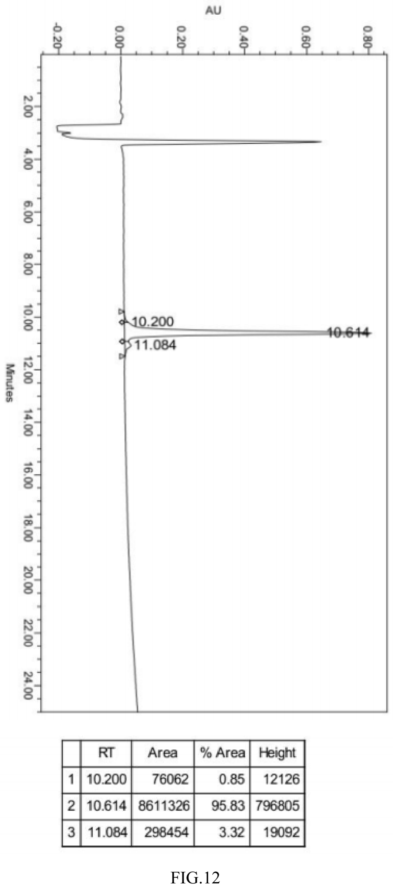

The mass spectra of SEQ ID NO.1-6 in this example are shown in FIGs. 1-6, and the HPLC chromatograms are shown in FIGs. 7-12.

### Experimental Example 1 BLI Affinity Assay

The BLI affinity assay was used to verify the effects of the cyclic peptides of the disclosure and the control product.

BLI refers to Bio-Layer Interferometry. The principle is: after visible light is emitted from the spectrometer, two reflected spectra are formed at the two interfaces of the optical film layer at the end of the sensor, and an interference spectrum is formed. Any change in film thickness caused by molecular binding can be reflected by the displacement value of the interference spectrum.

The His-tag recombinant human C3 protein was loaded onto a Ni-NTA biosensor (Sino Biological Inc.), and then association curves were generated with 7 cyclic peptides (SEQ ID NO.1-6, and the positive control group was the cyclic peptide Ac-ICVW(CH₃)QDWGAHRCT-NH₂ disclosed in the prior art, SEQ ID NO.7) (ForteBio Octet). Subsequently, a 90s dissociation step was performed after the 60s association phase to obtain the Kd value by fitting the curve.

The stock solution was dissolved in 15% acetonitrile, and the working solution was serially diluted with PBS at pH 8.0.

The experimental results are shown in Table 1 below:

**Table 1**

| NO. | Kd (M) | kon(1/Ms) | kdis(1/s) |
|---|---|---|---|
| 1 | 3.19E-06 | 1.54E+04 | 4.92E-02 |
| 2 | 4.54E-08 | 3.93E+05 | 1.79E-02 |
| 3 | 4.54E-08 | 3.87E+05 | 1.76E-02 |
| 4 | 3.51E-07 | 1.12E+05 | 3.93E-02 |
| 5 | 2.26E-06 | 1.31E+04 | 2.96E-02 |
| 6 | 3.99E-05 | 9.25E+02 | 3.69E-02 |
| 7 | 9.29E-08 | 2.02E+05 | 1.88E-02 |

The results showed that the Kd values of the cyclic peptides shown in SEQ ID NO.1-6 of the disclosure in the BLI affinity assay were all less than 1000 nM, the association rate constant kon was all greater than 1000 1/Ms, and the dissociation rate constant kdis was all less than 0.05 1/Ms.

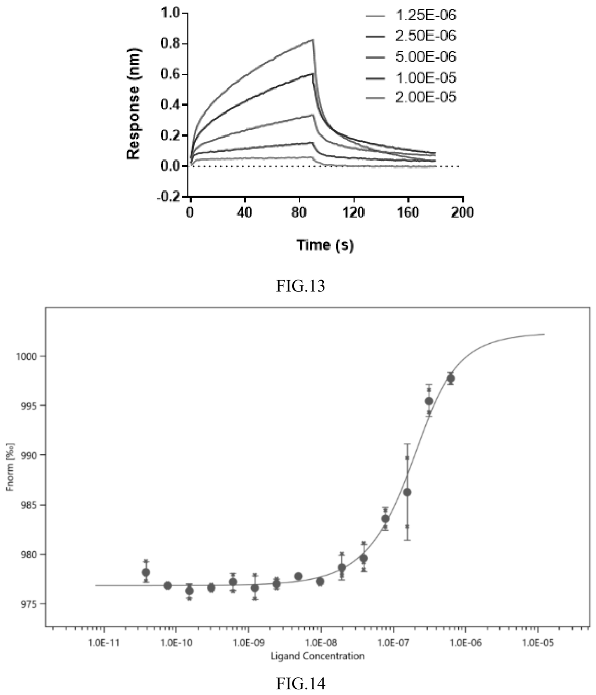

The real-time binding curves of each sample are shown in FIG. 13.

The results indicated that all test compounds had C3 protein affinity, and the most active one had a Kd value of <100 nM.

### Experimental Example 2 MST Affinity Assay

In this experimental example, the MST affinity assay was used to determine the Kd value of the binding between the cyclic peptide and C3 protein. The experiment was entrusted to Nanjing Zhongding Biotechnology Co., Ltd.

### 1. Reagents and Consumables:

1) Fluorescent labeling kit: Monolith Protein Labeling Kit RED-NHS 2nd Generation Kit;
2) C3 protein buffer: PBS;
3) Cyclic peptide buffer: 0.5×PBS, 15% ACN;
4) 20% Tween-20;
5) MST NT115 standard capillary.

### 2. Experimental Equipment

| Instrument Name | Manufacturer | Article number |
|---|---|---|
| Clean Bench | Suzhou Purification Equipment Co., Ltd. | SW-CJ-2F |
| MST Microscale Thermophoresis Instrument | NanoTemper, Germany | Monolith NT.115 |

| Constant Temperature Incubator | Shanghai CIMO Medical Instrument Co., Ltd | MJ-300B5H-III |
|---|---|---|
| Centrifuge | Hunan Xiangyi Laboratory Instrument Development Co., Ltd. | H1750R |

### 3. Experimental Methods and Results

In a standard labeled MST experiment, up to 16 dilution gradients can be measured at one time. The concentration of the fluorescently labeled molecule (Target, labeled molecule, cyclic peptide) is kept constant, while the other molecule (ligand, unlabeled molecule, C3 protein) is serially diluted 2-fold.

### 3.1 Ligand Molecule Dilution and Loading

10 µL of serially diluted unlabeled molecule (Ligand) and 10 µL of labeled molecule (Target) at a fixed concentration were mixed and allowed to react for 5 minutes. The mixed sample was loaded into a glass capillary and then analyzed by the MST-NT.115 instrument.

### 3.2 Pretest and Binding Check

Before the experiment, a Pretest step was performed to detect the fluorescence labeling intensity of the Target, detect protein adsorption and aggregation, and determine the experimental parameters.

### 3.3 Binding Affinity

After completing the Binding Check, if the protein had no adsorption or aggregation and the signal-to-noise ratio met the standard, the Binding Affinity assay was started.

Binding Affinity experimental parameters: the concentration of the three polypeptides on the machine was about 200 nM, Buffer (0.5×PBS, 15% ACN), and the maximum protein concentration was 0.25 µM with 2-fold serial dilution.

If the small molecule is soluble in an aqueous solution, the small molecule stock solution is directly serially diluted 2-fold 16 times. Each diluted solution is 10 µL, then 10 µL of the diluted protein solution is added and mixed. After aspiration with a capillary, it is placed in the instrument for detection. In this experimental case, the small molecule (protein) was dissolved in PBS. After serial dilution, 10 µL of the diluted Target solution was added to each tube, gently pipetted several times to mix, then aspirated with a capillary and placed in the instrument for analysis.

### 3.4 Experimental Results

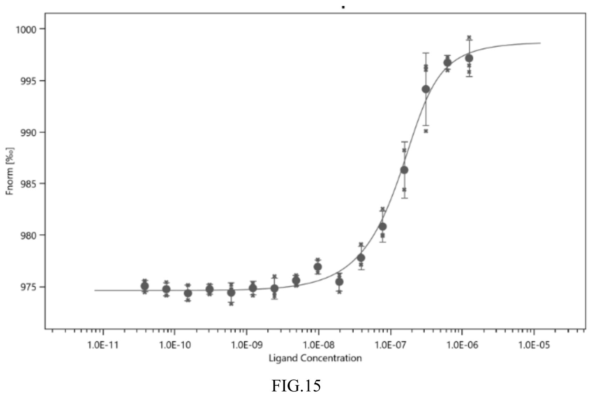

The results are shown in FIG. 14-FIG. 15. FIG. 14 is the fitting curve of SEQ ID NO.2, and FIG. 15 is the fitting curve of the positive control.

**Table 2**

| No. | 2 | 3 | 7 |
|---|---|---|---|
| Kd (M) | 1.1412E-07 | 3.5394E-08 | 1.2012E-07 |
| Kd (retest) | 2.4602E-08 | -- | 2.7311E-08 |

The results showed that the cyclic peptides shown in SEQ ID NO.1-6 of the disclosure all showed affinity in the BLI affinity assay, and the optimal Kd value was less than 100 nM. A larger Kd value indicates lower affinity, and vice versa, a smaller Kd value indicates higher affinity.

### Experimental Example 3 Solubility Assay

The synthesized SEQ ID NO.1-6 products and the positive control were dissolved in PBS to determine the solubility.

The solubility assay was performed with reference to the following steps:
Peptides (5-10 mg) were weighed in LoBind-Ependorf tubes; 20 µL of PBS (pH 7.4) was added, and the mixture was vortexed and centrifuged at 16873 g for 2 minutes. Unless otherwise stated, if the corresponding peptide was not dissolved, additional PBS was added in 10 µL increments, followed by vortexing and centrifugation until all precipitates disappeared. The pH of the peptide solution was measured on pH indicator strips or using a SevenExcellence pH meter (Mettler Toledo, Columbus, OH). The concentration of the final solution was determined based on the absorbance measured on a NanoDrop 2000c spectrophotometer as described above.

### Experimental Example 4 Determination of Half-Inhibitory Concentration Value

The half-inhibitory concentration values of SEQ ID NO.1-6 were evaluated by the classical pathway complement inhibition assay. The association constant (Ka), dissociation constant (Kd), and equilibrium dissociation constant (KD) were determined by surface plasmon resonance (SPR) assay.

The complement inhibition assay can be performed with reference to the following steps:
Evaluation was performed by an established enzyme-linked immunosorbent assay (ELISA). Under normal ambient temperature, 96-well plates were coated with 50 µL of 1% ovalbumin in PBS for 2 hours. The wells were blocked with 200 µL of 1% BSA in PBS for 1 hour, and then coated with 50 µL of 1:1000 diluted α-ovalbumin polyclonal antibody in PBS for 1 hour. Between each step, the plates were washed three times with 200 µL of 0.05% Tween 20 in PBS (PBS-T). Serial dilutions of cyclic peptides prepared in veronal buffer (VBS; 5 mM veronal, pH 7.4, containing 150 mM NaCl, 0.5 mM CaCl₂, and 0.5 mM MgCl₂) were added to the 96-well plates, followed by human plasma (1:40 in VBS), and the mixture was incubated at room temperature for 15 minutes. After washing, complement activation (C3b/iC3b/C3d bound to immune complexes) was detected using HRP-conjugated goat α-human C3 antibody (1:1000; MP Biomedicals). The reaction was performed using HRP substrate (0.05% ABTS and 0.1% H₂O₂ dissolved in 0.1 M sodium citrate, pH 4.2), and the absorbance was read at 405 nm using a VICTOR multi-label plate reader (PerkinElmer, Waltham, MA). 100% complement activation was obtained in the absence of the C3 peptide inhibitor, and the absorbance data obtained at 405 nm were converted to the percentage of complement inhibition. The percentage of inhibition was plotted against the logarithm of concentration, and the resulting data set was fitted to the equation "log (inhibitor) vs normalized response" using GraphPad Prism 5. IC50 values were obtained from the fitting parameters of the mean values from three independent experiments. Surface plasmon resonance (SPR) experiments were performed using a Biacore 3000 instrument (GE Healthcare, Piscataway Town, NJ), and the binding affinity and kinetic characteristics of the cyclic peptides were evaluated by SPR.

The experimental results are shown in Table 3 below:

**Table 3**

| No. | 2 | 3 | 4 | 7 |
|---|---|---|---|---|
| IC50 (µm) | 0.920 | 1.457 | 2.256 | 1.007 |

### Experimental Example 5 In Vivo Pharmacokinetic Evaluation

SEQ ID NO.1-6 and the positive control were administered systemically or locally to experimental animals, and samples were collected at different time points. To determine the pharmacokinetic parameters, evaluation can be performed by UPLC-ESI-MS analysis of the samples. The C3 or other marker levels of each animal were quantitatively determined. The total concentration of the parent compound and cleavage products was quantitatively determined.

In the disclosure, the evaluation of pharmacokinetics is not limited to the form presented in the experimental examples. Evaluation methods or indicators known to those skilled in the art can be used to evaluate the pharmacokinetic properties of the inhibitor provided by the disclosure.

The above examples are only presented as some preferred embodiments of the disclosure and are not intended to limit the disclosure. Conventional substitutions or equivalent substitutions made by those skilled in the art based on the technical solutions disclosed in the present application are all within the scope of protection of the disclosure.

## Claims

1. A complement inhibitor, wherein an active ingredient of the complement inhibitor comprises a cyclic peptide or a pharmaceutically acceptable salt of the cyclic peptide, wherein the cyclic peptide is shown in a general formula I: R1-IC-Xaa₃-Xaa₄-QD-Xaa₇-G-Xaa₉-HRCT-R2;
Xaa₇ and Xaa₉ are selected from uncommon amino acids, at least one of Xaa₃ or Xaa₄ is an uncommon amino acid;
R1 and R2 are modifying groups;
the general formula I is cyclized via a disulfide bridge formed between Cys-2 and Cys-12.

2. The complement inhibitor according to claim 1, wherein Xaa₃ is selected from common amino acids or uncommon amino acids; Xaa₄ is selected from uncommon amino acids; Xaa₇ is selected from either 2Nal or 5F-Trp; Xaa₉ is selected from either Aib or Pip.

3. The complement inhibitor according to claim 2, wherein Xaa₃ is selected from any one of V, Y, or Abu; Xaa₄ is selected from any one of 2Nal, W(CH₃), Bpa, or AzaTrp; Xaa₇ is selected from either 2Nal or 5F-Trp; Xaa₉ is selected from either Aib or Pip.

4. The complement inhibitor according to claim 1, wherein R1 is selected from any one of hydrogen, acetyl, methoxy, PEG or carboxyl, and R2 is selected from amino.

5. The complement inhibitor according to claim 4, wherein R1 is selected from acetyl group, and R2 is selected from amino group.

6. The complement inhibitor according to claim 1, wherein R1 and R2 are selected from amino acids or variants of amino acids.

7. The complement inhibitor according to claim 6, wherein R1 and R2 are independently selected from tyrosine or isoleucine respectively.

8. The complement inhibitor according to claim 7, wherein R1 is selected from isoleucine, and R2 is selected from tyrosine.

9. The complement inhibitor according to any one of claims 6-8, wherein R1 and R2 form an amide bond to loop polypeptide from end to end.

10. The complement inhibitor according to claim 1, wherein the active ingredient of the complement inhibitor is selected from one or more of SEQ ID NO.1- SEQ ID NO.6.

11. The complement inhibitor according to claim 1, wherein the active ingredient of the complement inhibitor is an optimized product with the general formula I; the optimization comprises one or more of PEGylation, conjugation, homo-multimerization, hetero-multimerization, amino acid chirality optimization, incorporation of hydrophilic/charged residues, addition of cell-penetrating peptides, and in-chain N-methylation.

12. The complement inhibitor according to claim 11, wherein the optimization occurs at R1 or R2 or backbone amino acids; preferably, the optimization is the incorporation of hydrophilic/charged residues at R2, and more preferably, the optimization is the incorporation of one or more K or R residues.

13. The complement inhibitor according to claim 11, wherein the PEGylation occurs at R1; and the in-chain N-methylation occurs at Xaa₉.

14. A method for preparing the complement inhibitor according to any one of claims 1-13 based on the amino acid sequence.

15. Use of the complement inhibitor according to any one of claims 1-13 in preparation of a drug for treating complement-mediated disorders.

16. The use according to claim 15, wherein the complement-mediated disorders comprise any one or a combination of ophthalmic diseases, glomerulonephritis, systemic lupus erythematosus, streptococcus pneumoniae infection, skin rashes, inflammation, paroxysmal nocturnal hemoglobinuria, atypical haemolytic uraemic syndrome, complement-mediated hemolysis, adult respiratory distress syndrome, heart diseases, or complement-mediated injury; the inflammation comprises periodontitis; the ophthalmic diseases comprise any one or a combination of macular degeneration, choroidal neovascularization, retinal neovascularization, and geographic atrophy; the drug is preferably an injection, more preferably a local injection, and most preferably a subcutaneous injection or intravenous injection; the drug is preferably an ophthalmic preparation, more preferably an intravitreal injection or ophthalmic drops.

17. A drug comprising the complement inhibitor according to any one of claims 1-13, or a salt, tandem, or multimer of the complement inhibitor.

18. The drug according to claim 17, wherein the drug comprises one or more of SEQ ID NO.1-SEQ ID NO.6.
